# NEUE EUROPÄISCHE PATENTSCHRIFT

(11) **EP 1 779 839 B2**
(45) Veröffentlichungstag und Bekanntmachung des Hinweises auf die Entscheidung über den Einspruch: **14.08.2019**
(45) Hinweis auf die Patenterteilung: 25.05.2011
(21) Anmeldenummer: 06122723.7
(22) Anmeldetag: 23.10.2006
(51) Int. Cl.: A61K 8/34, A61K 8/49, A61Q 17/04

(54) **Kosmetische Zubereitung mit 1,2-Alkandiol(e) und Triazin(en)**
Cosmetic composition comprising 1,2-alkanediol(s)and triazine(s)
Composition cosmétique comprenant 1,2-alkanediol(s) et triazine(s).

(30) Priorität: 25.10.2005 DE 102005051858
(43) Veröffentlichungstag der Anmeldung: 02.05.2007
(60) Teilanmeldung: 08103575.0
(73) Patentinhaber: Beiersdorf AG, 20253 Hamburg (DE)
(72) Erfinder: Behrens, Svea, 21109, Hamburg (DE); Bleckmann, Andreas, 22926, Ahrensburg (DE); Clausen, Andreas, 25436 Tornesch (DE); Meiring, Uta, 21077, Hamburg (DE); Nielsen, Jens, 25448, Henstedt-Ulzburg (DE)

(56) Entgegenhaltungen:
- EP-A- 1 078 638
- EP-A- 1 426 029
- WO-A1-00/78277
- WO-A2-2004/110366
- DATABASE EPODOC EUROPEAN PATENT OFFICE, THE HAGUE, NL; JP2000178162 27. Juni 2000 (2000-06-27), POLA CHEM IND INC: "Cosmetic" XP002413387 & JP 2000 178162 A (POLA CHEM IND INC) 27. Juni 2000 (2000-06-27)
- IP.com Journal, IPCOM000125183
- IP.com Journal, IPCOM000031257

## Beschreibung

Die vorliegende Erfindung betrifft eine kosmetische Zubereitung enthaltend ein oder mehrere 1,2-Alkandiole sowie ein oder mehrere UV-Lichtschutzfilter aus der Gruppe der Triazinderivate.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Eine wichtige Gruppe von UV-Lichtschutzfiltern baut auf dem Strukturelement des Triazins auf.

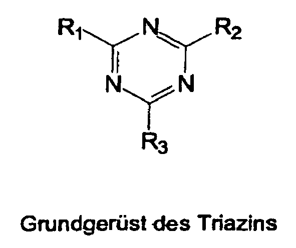

UV-Lichtschutzfilter, die das Grundgerüst des Triazins aufweisen (Triazinderivate) haben den Nachteil, dass sie in kosmetischen Zubereitungen nur schwer löslich sind. Dieser Sachverhalt führt insbesondere dann zu Problemen, wenn Zubereitungen mit einem hohen Lichtschutzfaktor hergestellt werden sollen, da in diesen Fällen nur ungenügende Mengen an Triazinderivaten in die Zubereitung eingearbeitet werden können.

Es war daher die Aufgabe der vorliegenden Erfindung Zubereitungen zu entwickeln, in die größere Mengen an Triazinderivaten eingearbeitet werden können. Insbesondere war es die Aufgabe der vorliegenden Erfindung, Zubereitungen mit einem hohen Lichtschutzfilterfaktor zu entwickeln. Diese Zubereitungen sollten, aufgrund des auf die Schutzbedürfnisse der menschlichen Haut angepassten Absorptionsspektrums von Triazinderivaten, einen besonders hohen Anteil an dieser UV-Filterklasse aufweisen.

Überraschend gelöst werden die Aufgaben durch eine kosmetische Zubereitung in Form einer Emulsion gemäß Anspruch 1.

Zwar beschreiben die EP 1 238 651 und die EP 1 078 638 Zubereitungen mit Lichtschutzfiltern und Alkandiolen, doch konnten diese Schriften nicht den Weg zur vorliegenden Erfindung weisen. Darüber hinaus kennt der Fachmann die EP 1 426 029, WO 2004/110366 und JP 2000-178162, die sich mit Kombinationen aus UV-Filtern und Diolen beschäftigen sowie die EP 1 034 778, US 2004/0151677 und EP 0 685 223, die sich mit der Löslichkeit von Triazinderivaten befassen. Auch diese Schriften konnten jedoch nicht den Weg zur vorliegenden Erfindung weisen.

Es ist erfindungsgemäß von Vorteil, wenn die erfindungsgemäße Zubereitung ein oder mehrere 1,2-Alkandiole in einer Gesamtkonzentration von 0,3 bis 3,0 Gewichts-%, bezogen auf das Gesamtgewicht der Zubereitung, enthält.

Es ist erfindungsgemäß bevorzugt, wenn die erfindungsgemäße Zubereitung ein oder mehrere 1,2-Alkandiole in einer Gesamtkonzentration von 0,5 bis 2,0 Gewichtes-%, bezogen auf das Gesamtgewicht der Zubereitung, enthält.

Der Begriff "erfindungsgemäß" bezieht sich im Rahmen dieser Offenbarung sowohl auf die erfindungsgemäße Zubereitung als auch auf die erfindungsgemäße Verwendung. Entsprechend gilt der Begriff "erfindungsgemäße Zubereitung" auch für die erfindungsgemäßen Verwendungen.

Es ist vorteilhaft im Sinne der vorliegenden Erfindung, wenn die Zubereitung ein oder mehrere UV-Lichtschutzfilter aus der Gruppe der Triazinderivate in einer Gesamtkonzentration von 0,3 bis 3,0 Gew.-%, bezogen auf das Gesamtgewicht der Zubereitung, enthält.

Es ist bevorzugt im Sinne der vorliegenden Erfindung, wenn die Zubereitung ein oder mehrere UV-Lichtschutzfilter aus der Gruppe der Triazinderivate in einer Gesamtkonzentration von 0,5 bis 2,0 Gew.-%, bezogen auf das Gesamtgewicht der Zubereitung, enthält.

Vorteilhafte Ausführungsformen der vorliegenden Erfindung sind dadurch gekennzeichnet, dass das Gewichtsverhältnis der Gesamtmenge an 1,2-Alkandiolen zur Gesamtmenge an UV-Lichtschutzfiltern aus der Gruppe der Triazinderivate in der Zubereitung von 2:1 bis 1:2, besonders bevorzugt 1:1 beträgt.

In erfindungsgemäßen Ausführungsformen der vorliegenden Erfindung werden als UV-Lichtschutzfilter aus der Gruppe der Triazinderivate ein oder mehrere Verbindungen aus der Gruppe der folgenden Verbindungen gewählt:
- 2,4-Bis-{[4-(2-ethyl-hexyloxy)-2-hydroxy]-phenyl}-6-(4-methoxyphenyl)-1,3,5-triazin (INCI: Aniso Triazin, erhältlich unter dem Handelsnamen Tinosorb S),
- Dioctylbutylamidotriazon (INCI: Dioctylbutamidotriazone),
- 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0, erhältlich bei 3V Sigma unter der Handelsbezeichnung Uvasorb® K2A),
- 2,4,6-Tribiphenyl-4-yl-1,3,5-triazin.

Darüber hinaus kann die erfindungsgemäße Zubereitung vorteilhaft weitere UV-Lichtschutzfilter enthalten. Vorteilhaft sind dabei insbesondere die nach der Kosmetikverordnung zugelassenen UV-Lichtschutzfilter. Derartige zusätzliche Filter können erfindungsgemäß vorteilhaft in Konzentrationen von 0,01 bis 30 Gew.-%, bezogen auf das Gesamtgewicht der Zubereitung in dieser enthalten sein. Dabei ist es erfindungsgemäß bevorzugt, wenn ein oder mehrere weitere UV-Lichtschutzfilter gewählt werden aus der Gruppe Phenylen-1,4-bis-(2-benzimidazyl)-3,3'-5,5'-tetrasulfonsäuresalze, 2-Phenylbenzimidazol-5-sulfonsäuresalze,1,4-di(2-oxo-10-Sulfo-3-bornylidenmethyl)-Benzol und dessen Salze, 4-(2-Oxo-3-bornylidenmethyl)benzolsulfonsäuresalze, 2-Methyl-5-(2-oxo-3-bornylidenmethyl)sulfonsäuresalze, 2,2'-Methylen-bis-(6-(2H-benzotriazol-2-yl)-4-(1,1,3,3-tetramethylbutyl)-phenol), 2-(2H-benzotriazol-2-yl)-4-methyl-6-[2-methyl-3-[1,3,3,3-tetramethyl-1-[(trimethylsilyl)oxy]disiloxanyl]propyl]-phenol, 3-Benzylidencampher; 4-(Dimethylamino)-benzoesäure(2-ethylhexyl)ester, 4-(Dimethylamino)benzoesäure-amylester; 4-Methoxybenzalmalonsäuredi(2-ethylhexyl)ester; Dioctylbutylamidotriazon, 4-Methoxyzimtsäure(2-ethylhexyl)ester, 4-Methoxyzimtsäureisopentylester; 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydoxybenzoyl)-benzoesäurehexylester 4-(tert.-Butyl)-4'-methoxydibenzoylmethan, Homomenthylsalicylat, 2-Ethylhexyl-2-hydroxybenzoat, 2-Ethylhexyl-2-cyano-3,3-diphenylacrylat, 3-(4-(2,2-bis Ethoxycarbonylvinyl)-phenoxy)propenyl)-methoxysiloxan / Dimethylsiloxan - Copolymer, Titandioxid, Zinkoxid.

Erfindungsgemäß ist die erfindungsgemäße Zubereitung frei von 3-(4-Methylbenzyliden)campher.

Es ist erfindungsgemäß, wenn die kosmetische Zubereitung in Form einer Emulsion vorliegt. Dabei ist es erfindungsgemäß bevorzugt, wenn die Zubereitung in Form einer O/W-Emulsion vorliegt. Erfindungsgemäß bevorzugt sind derartige Emulsionen frei von Natriumdihydroxycetylphosphat (Sodium Dihydroxycetyl Phosphate, z.B: Dragophos S).

Es ist erfindungsgemäß vorteilhaft, wenn die erfindungsgemäße Zubereitung Tocopherylacetat enthält. Tocopherylacetat ist erfindungsgemäß vorteilhaft in einer Konzentration von 0,1 bis 1,0 Gewichts-% und bevorzugt in einer Konzentration von 0,3 bis 0,7 Gewichts-%, bezogen auf das Gesamtgewicht der Zubereitung, in der Emulsion enthalten.

Die erfindungsgemäße Zubereitung kann erfindungsgemäß vorteilhaft als Salbe, Creme oder Lotion (ggf. sprühbar) vorliegen. Die erfindungsgemäße Zubereitung kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Emulsion, erfindungsgemäß.

Die Wasserphase der erfindungsgemäßen Zubereitung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner (z.B. Dihydroxyaceton), Repellentien sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die Ölphase der erfindungsgemäßen Zubereitung kann alle herkömmlichen Bestandteile von in der Kosmetik eingesetzten Öl-, Fett- und Wachskomponenten enthalten.

Die erfindungsgemäße Zubereitung kann erfindungsgemäß vorteilhaft kosmetische Wirk- und Pflegestoffe enthalten, beispielsweise die nach der Kosmetikverordnung zugelassenen Konservierungsmittel oder Konservierungshelfer. Als weitere Pflegestoffe können insbesondere Niacinamid, Panthenol, Aloe vera, Hammamelis-Extrakt, Polidocanol, Vitamin E, Vitamin A, Vitamin A-Derivate, Vitamin C, Vitamin C-Derivate, Coenzym Q10, Kreatin, Taurin, Alphaglycosylrutin und/oder in Konzentrationen von 0,1 bis 30 Gew.-%, bezogen auf das Gesamtgewicht der Zubereitung eingesetzt werden. Derartige Wirkstoffe können erfindungsgemäß vorteilhaft in Konzentrationen von 0,01 bis 30 Gew.-%, bezogen auf das Gesamtgewicht der Zubereitung in dieser enthalten sein.

Die erfindungsgemäßen Zubereitungen sind erfindungsgemäß bevorzugt frei von lodopropynylbutylcarbamat.

Erfindungsgemäß besonders bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gew.-%, bezogen auf das Gesamtgewicht der Zubereitung eingesetzt.

Die nachfolgenden Beispiele sollen die vorliegende Erfindung verdeutlichen ohne sie einzuschränken. Die Angaben beziehen sich stets auf Gew.-%, sofern nicht andere Angaben gemacht werden.

**Beispiele (O/W-Emulsionen)**

| **Beispiel** | **1** | **2** | **3** | **4** | **5** | **6** | **7** |
|---|---|---|---|---|---|---|---|
| Glyceryl Stearate Citrat | | 1,0 | | 2,0 | | | 2,0 |
| PEG-40 Stearat | 1,0 | | | | 1,5 | | |
| Polyglycerylmethylglucose-distearat | | | 2,0 | | | 1,5 | 0,5 |
| Glycerylstearate | 1,5 | | | | 1,5 | | |
| Cetyl Alkohol | | 0,5 | | 2,0 | 0,75 | 1,0 | |
| Stearyl Alcohol | | 0,5 | 0,5 | | 0,75 | | |
| Cetearyl Alkohol | 2,0 | | | | | | 1,5 |
| Caprylic/Capric Triglycerid | 5,0 | 4,0 | | 5,0 | 6,0 | 3,0 | 0,5 |
| Ethylhexylcocoat | | | 2,0 | | | 1,0 | |
| Octyldodecanol | | 1,0 | 3,0 | | | 2,0 | |
| Mineral Oil | | | 2,0 | | | | |
| Hydriertes Polyisobuten | 1,0 | | | | | | |
| Polydecen | | | | 2,0 | | | |
| Cyclomethicon | 2,0 | | | | 3,0 | | |
| Dimethicon | 1,0 | | | | | | 1,5 |
| Phenyltrimethicon | | | | | 1,0 | | |
| Dicaprylyl Carbonat | | 2,0 | | | | | 3,5 |
| Natürliche Öle (wie z.B. Jojobaöl / Sonnenblumenöl) | 1,5 | | 3,0 | 0,5 | | 2,0 | 2,5 |
| **1,2 Hexandiol** | **0,5** | **0,75** | **3,0** | **0,3** | **1,5** | **1,0** | **0,75** |
| Aniso Triazin | 0,5 | 0,25 | 1,5 | 0,3 | 2,0 | 0,75 | 0,5 |
| Octyl Triazone | 0,5 | 0,5 | 1,5 | 0,2 | | 0,5 | 1,0 |
| Trisodium EDTA | 0,2 | 0,1 | | 0,05 | 0,1 | 0,3 | |
| Iminodisuccinat | 0,1 | | 0,1 | | | | 0,5 |
| Phenoxyethanol | 0,3 | 0,1 | | 0,5 | | | 0,4 |
| Parabene | 0,4 | | 0,3 | | | 0,2 | |
| Hexamidin Diisethionat | | 0,1 | | | | 0,1 | |
| Imidodiazolydynl Urea | | | | | 0,2 | | 0,2 |
| DMDM Hydantoin | | | 0,2 | | 0,1 | | |
| lodopropynyl Butylcarbamat | | | | 0,2 | | 0,05 | |
| Glycerin | 10,0 | 3,0 | 7,0 | 8,0 | 20,0 | 0,5 | 2,0 |
| Tocopherylacetat | 0,2 | 0,5 | 0,75 | | 0,3 | | 1,0 |
| Alcohol Denat. | 5,0 | | 2,5 | | 7,5 | | 7,5 |
| Wasser | ad.100 | ad100 | ad100 | ad100 | ad100 | ad100 | ad100 |

## Patentansprüche

1. Kosmetische Zubereitung in Form einer Emulsion enthaltend
a) 1,2-Hexandiol und
b) ein oder mehrere UV-Lichtschutzfilter aus der Gruppe 2,4-Bis-{[4-(2-ethyl-hexyloxy)-2-hydroxy]-phenyl}-6-(4-methoxyphenyl)-1,3,5-triazin, Dioctylbutylamidotriazon, 2,4-bis-[5-1(dimethylpropyl)benzoxazol-2-yl-(4-phenyl)-imino]-6-(2-ethylhexyl)-imino-1,3,5-triazin, 2,4,6-Tribiphenyl-4-yl-1,3,5-triazin, wobei die Zubereitung **dadurch gekennzeichnet ist, dass** sie frei ist von 3-(4-Methylbenzyliden)campher.

2. Kosmetische Zubereitung nach Anspruch 1, **dadurch gekennzeichnet, dass** die Zubereitung ein oder mehrere 1,2-Alkandiole in einer Gesamtkonzentration von 0,3 bis 3,0 Gewichts-%, bezogen auf das Gesamtgewicht der Zubereitung, enthält.

3. Kosmetische Zubereitung nach einem der vorhergehenden Ansprüche, **dadurch gekennzeichnet, dass** die Zubereitung ein oder mehrere UV-Lichtschutzfilter aus der Gruppe der Triazinderivate in einer Gesamtkonzentration von 0,3 bis 3,0 Gewichts-%, bezogen auf das Gesamtgewicht der Zubereitung, enthält.

4. Kosmetische Zubereitung nach einem der vorhergehenden Ansprüche, **dadurch gekennzeichnet, dass** das Gewichtsverhältnis der Gesamtmenge an 1,2-Alkandiolen zur Gesamtmenge an UV-Lichtschutzfiltern aus der Gruppe der Triazinderivate in der Zubereitung von 2:1 bis 1:2, besonders bevorzugt 1:1 beträgt.

5. Kosmetische Zubereitung nach einem der vorhergehenden Ansprüche, **dadurch gekennzeichnet, dass** die Zubereitung weitere UV-Lichtschutzfilter gewählt aus der Gruppe Phenylen-1,4-bis-(2-benzimidazyl)-3,3'-5,5'-tetrasulfonsäuresalze, 2-Phenylbenzimidazol-5-sulfonsäuresalze,1,4-di(2-oxo-10-Sulfo-3-bornylidenmethyl)-Benzol und dessen Salze, 4-(2-Oxo-3-bomylidenmethyl)benzolsulfonsäuresalze, 2-Methyl-5-(2-oxo-3-bomylidenmethyl)sulfonsäuresalze, 2,2'-Methylen-bis-(6-(2H-benzotriazol-2-yl)-4-(1,1,3,3-tetramethylbutyl)-phenol), 2-(2H-benzotriazol-2-yl)-4-methyl-6-[2-methyl-3-[1,3,3,3-tetramethyl-1-((trimethylsilyl)oxy]disiloxanyl]propyl]-phenol, 3-Benzylidencampher; 4-(Dimethylamino)-benzoesäure(2-ethylhexyl)ester, 4-(Dimethylamino)benzoesäure-amylester; 4-Methoxybenzalmalonsäuredi(2-ethylhexyl)-ester; Dioctylbutylamidotriazon, 4-Methoxyzimtsäure(2-ethylhexyl)ester, 4-Methoxyzimtsäureisopentylester; 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydoxybenzoyl)-benzoesäurehexylester 4-(tert.-Butyl)-4'-methoxydibenzoylmethan, Homomenthylsalicylat, 2-Ethylhexyl-2-hydroxybenzoat, 2-Ethylhexyl-2-cyano-3,3-diphenylacrylat, 3-(4-(2,2-bis Ethoxycarbonylvinyl)-phenoxy)propenyl)-methoxysiloxan / Dimethylsiloxan - Copolymer, Titandioxid, Zinkoxid, enthält.

6. Kosmetische Zubereitung nach einem der vorhergehenden Ansprüche, **dadurch gekennzeichnet, dass** die Zubereitung Tocopherylacetat enthält.

## Claims

1. Cosmetic preparation in the form of an emulsion comprising
a) 1,2-hexanediol and
b) one or more UV photoprotective filters from the group of 2,4-bis{[4-(2-ethylhexyloxy)-2-hydroxy]phenyl}-6-(4-methoxyphenyl)-1,3,5-triazine, dioctylbutylamidotriazone, 2,4-bis[5-1(dimethylpropyl)benzoxazol-2-yl-(4-phenyl)imino)-6-(2-ethylhexyl)imino-1,3,5-triazine, 2,4,6-tribiphenyl-4-yl-1,3,5-triazine, where the preparation is **characterized in that** it is free from 3-(4-methylbenzylidene)camphor.

2. Cosmetic preparation according to Claim 1, **characterized in that** the preparation comprises one or more 1,2-alkanediols in a total concentration of from 0.3 to 3.0% by weight, based on the total weight of the preparation.

3. Cosmetic preparation according to one of the preceding claims, **characterized in that** the preparation comprises one or more UV photoprotective filters from the group of triazine derivatives in a total concentration of from 0.3 to 3.0% by weight, based on the total weight of the preparation.

4. Cosmetic preparation according to one of the preceding claims, **characterized in that** the weight ratio of the total amount of 1,2-alkanediols to the total amount of UV photoprotective filters from the group of triazine derivatives in the preparation is from 2:1 to 1:2, particularly preferably 1:1.

5. Cosmetic preparation according to one of the preceding claims, **characterized in that** the preparation comprises further UV photoprotective filters selected from the group phenylene-1,4-bis(2-benzimidazyl)-3,3'-5,5'-tetrasulphonic acid salts, 2-phenylbenzimidazole-5-sulphonic acid salts, 1,4-di(2-oxo-10-sulpho-3-bornylidenemethyl)benzene and salts thereof, 4-(2-oxo-3-bornylidenemethyl)benzenesulphonic acid salts, 2-methyl-5-(2-oxo-3-bornylidenemethyl)sulphonic acid salts, 2,2'-methylenebis(6-(2H-benzotriazol-2-yl)-4-(1,1,3,3-tetramethylbutyl)phenol), 2-(2H-benzotriazol-2-yl)-4-methyl-6-[2-methyl-3-[1,3,3,3-tetramethyl-1-[(trimethylsilyl)oxy]-disiloxanyl]propyl]phenol, 3-benzylidenecamphor; 2-ethylhexyl 4-(dimethylamino)benzoate, amyl 4-(dimethylamino)benzoate; di(2-ethylhexyl) 4-methoxybenzalmalonate; dioctylbutylamidotriazone, 2-ethylhexyl 4-methoxycinnamate, isopentyl 4-methoxycinnamate; 2-hydroxy-4-methoxybenzophenone, 2-hydroxy-4-methoxy-4'-methylbenzophenone, 2,2'-dihydroxy-4-methoxybenzophenone, hexyl 2-(4'-diethylamino-2'-hydroxybenzoyl)benzoate, 4-(tert-butyl)-4'-methoxydibenzoylmethane, homomenthyl salicylate, 2-ethylhexyl 2-hydroxybenzoate, 2-ethylhexyl 2-cyano-3,3-diphenylacrylate, 3-(4-(2,2-bis-ethoxycarbonylvinyl)phenoxy)propenyl)methoxysiloxane/dimethylsiloxane copolymer, titanium dioxide, zinc oxide.

6. Cosmetic preparation according to one of the preceding claims, **characterized in that** the preparation comprises tocopheryl acetate.

## Revendications

1. Préparation cosmétique sous forme d'une émulsion, contenant
a) 1,2-hexanediol et
b) un ou plusieurs filtres de protection contre la lumière UV du groupe formé par la 2,4-bis-{[4-(2-éthylhexyloxy)-2-hydroxy]-phényl}-6-(4-méthoxyphényl)-1,3,5-triazine, la dioctylbutylamidotriazone, la 2,4-bis-[5-1(diméthylpropyl)benzoxazol-2-yl-(4-phényl)-imino]-6-(2-éthylhexyl)-imino-1,3,5-triazine, la 2,4,6-tribiphényl-4-yl-1,3,5-triazine,
la préparation étant **caractérisée en ce qu'**elle est exempte de 3-(4-méthylbenzylidène)camphre.

2. Préparation cosmétique selon la revendication 1, **caractérisée en ce que** la préparation contient un ou plusieurs 1,2-alcanediols en une concentration totale de 0,3 à 3,0% en poids, par rapport au poids total de la préparation.

3. Préparation cosmétique selon l'une quelconque des revendications précédentes, **caractérisée en ce que** la préparation contient un ou plusieurs filtres de protection contre la lumière UV du groupe des dérivés de triazine en une concentration totale de 0,3 à 3,0% en poids, par rapport au poids total de la préparation.

4. Préparation cosmétique selon l'une quelconque des revendications précédentes, **caractérisée en ce que** le rapport pondéral de la quantité totale de 1,2-alcanediols à la quantité totale de filtres de protection contre la lumière UV du groupe des dérivés de triazine dans la préparation est de 2:1 à 1:2, de manière particulièrement préférée de 1:1.

5. Préparation cosmétique selon l'une quelconque des revendications précédentes, **caractérisée en ce que** la préparation contient d'autres filtres de protection contre la lumière UV choisis dans le groupe formé par les sels de l'acide phénylène-1,4-bis-(2-benzimidazyl)-3,3'-5,5'-tétrasulfonique, les sels de l'acide 2-phénylbenzimidazole-5-sulfonique, le 1,4-di(2-oxo-10-sulfo-3-bornylidèneméthyl)-benzène et ses sels, les sels de l'acide 4-(2-oxo-3-bornylidèneméthyl)benzènesulfonique, les sels de l'acide 2-méthyl-5-(2-oxo-3-bornylidèneméthyl)sulfonique, le 2,2'-méthylènebis-(6-(2H-benzotriazol-2-yl)-4-(1,1,3,3-tétraméthylbutyl)-phénol), le 2-(2H-benzotriazol-2-yl)-4-méthyl-6-[2-méthyl-3-(1,3,3,3-tétraméthyl-1-[(triméthylsilyl)oxy]disiloxanyl]propyl]-phénol, le 3-benzylidènecamphre ; l'ester 2-éthylhexylique de l'acide 4-(diméthylamino)-benzoïque, l'ester amylique de l'acide 4-(diméthylamino)benzoïque ; l'ester di(2-éthylhexylique) de l'acide 4-méthoxybenzalmalonique ; la dioctylbutylamidotriazone, l'ester 2-éthylhexylique de l'acide 4-méthoxycinnamique, l'ester isopentylique de l'acide 4-méthoxycinnamique ; la 2-hydroxy-4-méthoxybenzophénone, la 2-hydroxy-4-méthoxy-4'-méthylbenzophénone, la 2,2'-dihydroxy-4-méthoxybenzophénone, l'ester hexylique de l'acide 2-(4'-diéthylamino-2'-hydoxybenzoyl)-benzoïque, le 4-(tert-butyl)-4'-méthoxydibenzoylméthane, le salicylate d'homomenthyle, le 2-hydroxybenzoate de 2-éthylhexyle, l'acrylate de 2-éthylhexyl-2-cyano-3,3-diphényle, le copolymère de 3-(4-(2,2-bis éthoxycarbonylvinyl)-phénoxy)propényl)-méthoxysiloxane/diméthylsiloxane, le dioxyde de titane, l'oxyde de zinc.

6. Préparation cosmétique selon l'une quelconque des revendications précédentes, **caractérisée en ce que** la préparation contient de l'acétate de tocophéryle.
